# FASCICULE DE BREVET EUROPEEN

(11) **EP 3 206 607 B1**
(45) Date de publication et mention de la délivrance du brevet: **16.05.2018**
(21) Numéro de dépôt: 15805562.4
(22) Date de dépôt: 15.10.2015
(51) Int. Cl.: A61B 17/72

(54) **CLOU CENTRO-MEDULLAIRE**
INTRAMEDULLÄRER NAGEL
INTRAMEDULLARY NAIL

(30) Priorité: 15.10.2014 FR 1459882; 03.02.2015 FR 1550832
(43) Date de publication de la demande: 23.08.2017
(73) Titulaire: 3 S ORTHO, 69009 Lyon (FR)
(72) Inventeur: PERRET, Jean-Pierre, 74300 Thiez (FR); SCARLAT, Marius, 83400 Hyeres (FR)
(74) Mandataire: Jeannet, Olivier
(86) Numéro de dépôt international: PCT/FR2015/052775
(87) Numéro de publication internationale: WO 2016/059347

(56) Documents cités:
- DE-A1-102009 030 177
- US-A1- 2007 123 878

## Description

La présente invention concerne un clou centro-medullaire, apte à être introduit dans un canal médullaire d'un os, destiné soit à la réduction d'une fracture de la tête ou du corps d'un os long d'un patient, soit à l'allongement osseux.

Dans le cas de réduction de fractures, un clou ou une tige centro-médullaire agit comme un tuteur interne partageant avec l'os la prise en charge des contraintes bio-mécaniques favorisant la consolidation osseuse.

Les clous centro-médullaires sont généralement utilisés pour la réduction de fractures du tibia, du fémur ou de l'humérus.

Ces clous sont particulièrement avantageux notamment pour la réduction de fractures de la tête d'un os permettant de rassembler et de consolider de multiples fragments fracturés.

Les clous centro-médullaires sont également utilisés dans l'allongement osseux de membres supérieurs et inférieurs. Ces clous d'allongement comprennent une partie coulissante, réglable par voie mécanique.

Un clou centro-médullaire est généralement en alliage de titane ou en acier inoxydable. Un implant en alliage de titane a la particularité de pouvoir combiner une stabilité mécanique avec des propriétés élastiques. En outre, l'avantage d'un implant en acier inoxydable réside notamment dans une résistance accrue à des contraintes mécaniques importantes.

Des vis, généralement auto-foreuses, maintiennent le clou aux parties d'os fracturés ou à allonger. Ces vis traversent de part en part le clou centro-médullaire par des orifices de fixation. Ces orifices de fixation sont taraudés par un filet simple, tel que dans le document EP 0 855 882 pour la réduction d'une fracture du fémur et les documents FR 2 881 340, EP 1 398 000, et US 5,472,444 pour la réduction d'une fracture d'un humérus.

Un clou centro-médullaire étant de faible diamètre, le pas de vis du taraudage est forcément étroit. Par conséquent, un certain nombre de tours de révolution sont nécessaires pour traverser le clou, ce qui exerce des pressions non négligeables sur les os et les tissus environnants.

Un taraudage à simple filet nécessite certaines opérations de rotation de la vis pour enclencher le vissage. Il n'est ainsi pas aisé de pouvoir enclencher, sans rotation préalable une vis dans un écrou à simple filet, au maximum, en réalisant un tour de révolution.

Lors des phases opératoires de mise en place des organes de fixation maintenant les os à consolider ou à allonger avec un clou centro-médullaire, l'engagement d'une vis à l'entré d'un orifice de fixation, avant toute opération de rotation, nécessite de position la vis selon un angle déterminé. L'engagement du filetage de la vis en correspondance du taraudage de l'orifice de fixation, nécessite des ajustements minutieux, voir un geste très précis, pour ne pas exercer des contraintes supplémentaires, voire traumatiques notamment pour les tissus environnants les fragments osseux. Le document US 2007/0123878 A1 divulgue un clou centro-médullaire selon le préambule de la revendication 1. La présente invention propose ainsi un clou centro-médullaire permettant de pallier aux inconvénients précités.

Ainsi, le clou centro-médullaire, apte à être agencé dans le canal médullaire d'un os long, selon l'invention, comprend un corps plein et au moins un orifice de fixation apte à coopérer avec un organe de fixation. L'orifice de fixation comprend un taraudage à au moins deux filets, tandis que le taraudage est réalisé sur une partie du diamètre du clou centro-médullaire, à savoir que le taraudage est entouré latéralement de part et d'autre par des conduits non taraudés.

Selon une caractéristique complémentaire, les conduits non taraudés sont rectilignes sur au moins une partie.

Ajoutons que le taraudage est avantageusement centré sur le diamètre du clou centro-médullaire.

Notons que la partie du diamètre du clou centro-médullaire, comprenant le taraudage, est comprise entre 20 et 80 % du diamètre, avantageusement entre 20 et 60 %, de préférence entre 20 et 40 %, plus préférentiellement le taraudage est réalisé sur environs le tiers du diamètre du clou centro-médullaire.

Ajoutons que l'orifice de fixation comprend préférentiellement un taraudage à trois filets.

Selon une caractéristique complémentaire, l'orifice de fixation comprend un taraudage avec un pas axial constant et un profil triangulaire.

Selon une autre caractéristique, la partie proximale comprend au moins deux orifices de fixation décalés angulairement les uns part à rapport aux autres selon un angle compris entre 20° et 50°, préférentiellement entre 30° et 40°.

Notons que l'orifice de fixation comprend un pas hélicoïdal compris entre 1 et 3 millimètre(s).

Selon une caractéristique complémentaire, l'orifice de fixation et/ou l'organe de fixation comprend un moyen de blocage de l'organe de fixation.

D'autres caractéristiques et avantages de l'invention se dégageront de la description qui va suivre en regard des dessins annexés qui ne sont donnés qu'à titre d'exemples non limitatifs.
Les figures 1 à 7 sont des vues d'un clou centro-médullaire huméral selon un mode de réalisation de l'invention.
La figure 1 est une vue de coté.
La figure 2 est une vue en coupe selon l'axe (A-A) de la figure 1.
La figure 3 est un grossissement de la vue en coupe selon l'axe (C-C) de la figure 2.
La figure 4 est une vue simplifiée de la figure 2 avec un organe de fixation.
La figure 5 est une vue en coupe selon (C-C) de la figure 4.
La figure 6 est un grossissement de la vue du détail (D) de la figure 5.
La figure 7 est une vue en perspective de l'entré d'un orifice de fixation.

Ainsi, le clou centro-médullaire (100) selon l'invention est un clou non canalaire, à savoir, qui comprend un corps plein constitué de manière connue par une partie proximale (1), une partie centrale (2) et une partie distale (3).

Notons que le clou centro-médullaire (100) de l'invention comprend des orifices de fixation (4) comprenant un taraudage à au moins deux filets, préférentiellement à trois filets, apte à recevoir des organes de fixation (5), tels que des vis, préférentiellement auto-foreuses.

Il est entendu qu'un taraudage à multiples filets correspond à de multiples taraudages parallèles entre eux.

L'orifice de fixation (4) selon l'invention comprend ainsi au moins deux taraudages, préférentiellement trois taraudages, décalés à l'entrée du dit orifice d'au moins 180°, préférentiellement de 120°, permettant l'entrée d'un organe de fixation au moins tous les demi-tours, préférentiellement tous les tiers de tours.

Cette configuration d'orifice de fixation (4) permet ainsi de limiter la manipulation par rotation de l'organe de fixation (5) au niveau de l'entrée du dit orifice de fixation (4).

Cette configuration permet également d'obtenir une plus grande avance pour un tour de vis en comparaison de celle d'un écrou à simple filet.

En effet, avec un taraudage à multiples filets, pour une même distance longitudinale parcourue, par rapport à un taraudage à simple filet, le mouvement de rotation est divisé par deux pour un taraudage à double filets et par trois pour un taraudage à triple filets.

Ainsi, lors de l'insertion d'une vis dans un écrou à simple filet, l'opérateur procède à une rotation d'au maximum 360°, alors qu'avec un écrou à deux ou trois filets, le chirurgien procède à une rotation d'au maximum 180° ou au maximum 120° respectivement.

Dans une optique d'optimisation du geste du chirurgien lors des opération d'insertion et de vissage, il est préféré un taraudage à triple filets.

La présente invention permet donc de limiter les manipulations d'insertion de l'organe de fixation (5) dans l'orifice de fixation (4), et donc de limiter la pression exercée sur le clou centro-médullaire (100) lors du placement de ce dernier, et par conséquent, de limiter la pression sur les os et les tissus environnants.

Il est entendu que le filet du taraudage forme une saillie hélicoïdale continue et de section uniforme sur une surface cylindrique ou conique, préférentiellement cylindrique.

Il est en outre entendu qu'un pas hélicoïdal (Ph) est la distance qui sépare deux sommets consécutifs d'un même filet.

Il est également entendu que dans le cadre de l'invention d'un taraudage à plusieurs filets, le pas (Pa) axial, correspondant à la distance entre deux sommets consécutifs, est égal au pas hélicoïdal (Ph) divisé par le nombre de filets.

Il est également entendu que pour qu'un écrou puisse être assemblé à une vis, les deux éléments doivent avoir les mêmes caractéristiques, telles que profil du filet, diamètre nominal (Dn), pas (Ph) et sens de l'hélice.

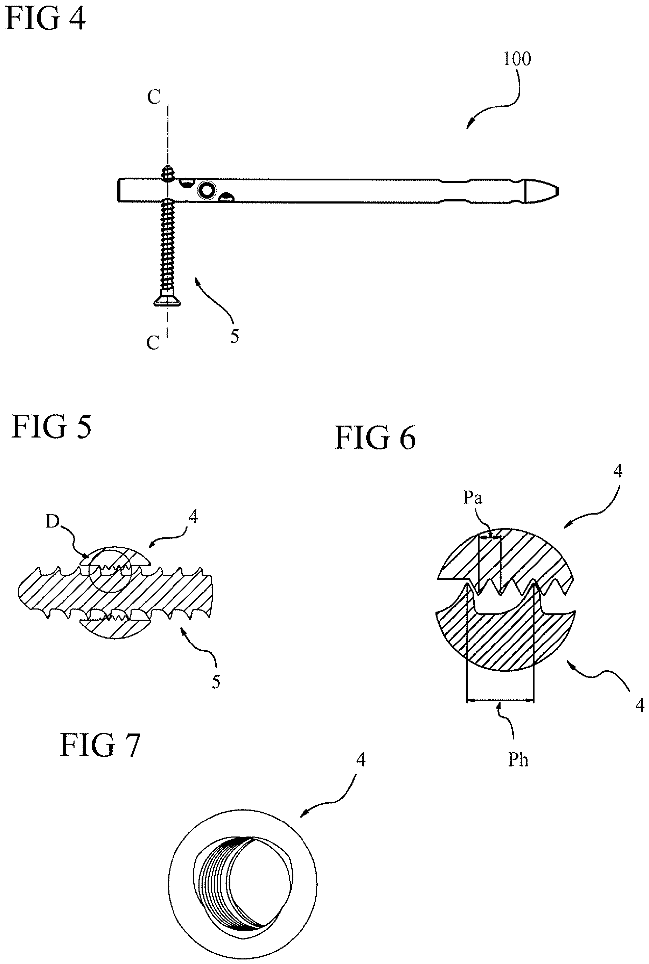

L'organe de fixation (5) a par conséquent un pas de filetage égal au pas hélicoïdale (Ph) de taraudage de l'orifice de fixation (4) tel qu'illustré aux figures 4 et 5.

Le pas hélicoïdal (Ph) d'un filet est compris entre 1 et 3 millimètre(s).

Le pas hélicoïdal (Ph) d'un filet est avantageusement fonction du diamètre correspondant de l'orifice de fixation (4).

Le pas (Pa) est avantageusement constant sur toute la longueur du taraudage de l'orifice de fixation (4).

Le taraudage a avantageusement un profil triangulaire avec un angle, avantageusement égal à 60°, mais il en pourrait être autrement, le filetage est avantageusement arrondi ou carré.

Les orifices de fixation (4), selon une caractéristique, sont au moins localisés en partie proximale (1) du clou centro-médullaire (100) pour notamment une réduction d'une fracture de la tête d'un os long d'un membre.

Selon la caractéristique précédente, le clou centro-médullaire (100) comprend au moins deux orifices de fixation (4) en partie proximale (1), préférentiellement un nombre d'orifices de fixation (4) compris entre 2 et 6.

Les orifices de fixation (4), selon une autre caractéristique, sont au moins localisés en partie centrale (2) et/ou en partie distale (3) du clou centro-médullaire (100) pour notamment une réduction d'une fracture du corps d'un os long d'un membre.

Les orifices de fixation (4) sont avantageusement localisés en partie proximale (1), en partie centrale (2) et en partie distale (3) du clou centro-médullaire (100) pour notamment une réduction d'une fracture, ou pour l'allongement d'un os long d'un membre.

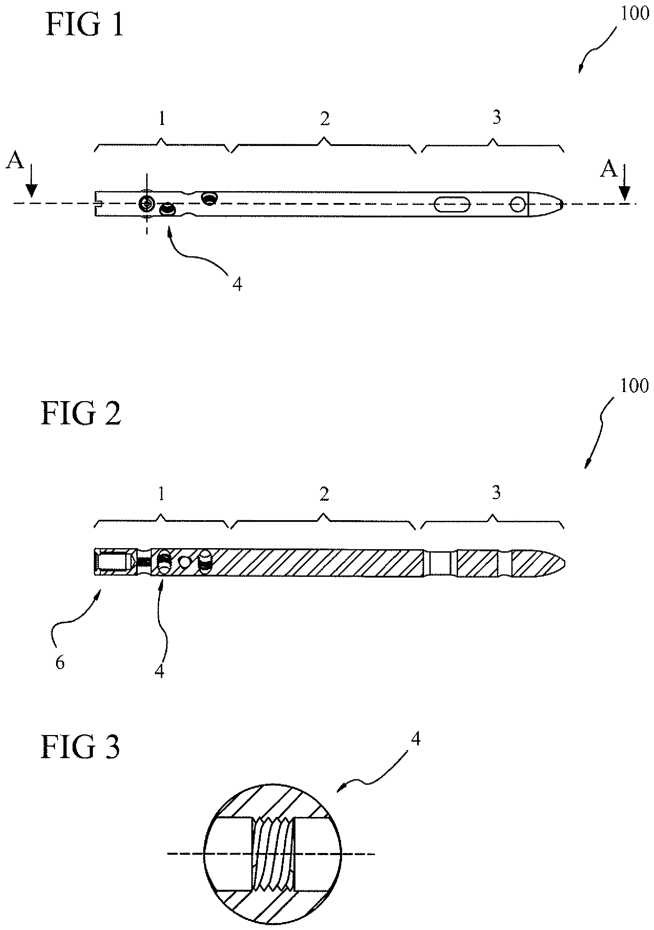

Selon un mode de réalisation illustré notamment à la figure 2, les orifices de fixation (4) sont décalés angulairement les un part à rapport aux autres selon un angle compris entre 20° et 50°, préférentiellement entre 30° et 40°, plus préférentiellement 35°.

Les angles entre les orifices de fixation (4) sont avantageusement égaux, mais il pourrait en être autrement.

Rappelons qu'afin de faciliter la fixation d'un organe de fixation (5) dans un orifice de fixation (4), le taraudage de ce dernier est un taraudage à au moins deux filets. Cependant, afin de faciliter l'engagement et le positionnement selon un axe commun d'un organe de fixation (5) dans un orifice de fixation (4), ce dernier comprend des systèmes auto-centreurs.

Ainsi, le taraudage d'un orifice de fixation (4) est réalisé sur une partie du diamètre du clou centro-médullaire (100), préférentiellement centré selon un axe longitudinal (A-A) médian du clou centro-médullaire (100), à savoir centré par rapport au diamètre du clou centro-médullaire (100).

Ajoutons qu'un taraudage est entouré latéralement de part et d'autre de systèmes auto-centreurs, à savoir de conduits non taraudés, préférentiellement rectiligne sur au moins une partie. Précisons que les conduits non taraudés sont au moins rectilignes à l'entré du taraudage.

Notons que la partie du diamètre du clou centro-médullaire, comprenant le taraudage, est comprise entre 20 et 80 %, avantageusement entre 20 et 60 %, de préférence entre 20 et 40 %, plus préférentiellement le taraudage est réalisé sur environs le tiers du diamètre, tel qu'illustré notamment à la figure 4.

Il est entendu que le diamètre d'un système auto-centreur, à savoir d'un conduit non taraudé, est supérieur ou égal à celui d'un organe de fixation (5), permettant son insertion dans l'orifice correspondant.

Précisons que le diamètre de la profondeur du taraudage est inférieur ou égal aux diamètres des conduits non taraudés.

Ainsi la combinaison des systèmes auto-centreurs et des taraudages à au moins deux filets, permettent un engagement et un visage des organes de fixations (5) dans les orifices de fixations (4) de manière optimale, limitant les contraintes exercer sur les os et les tissus environnants.

Selon des autres modes de réalisation, un orifice de fixation (4) et/ou un organe de fixation (5) comprend un moyen de blocage de l'organe de fixation (4), par exemple par des moyens de blocage tels que ceux décrits dans les documents EP1398000 et FR2881340, ou des moyens de blocage communément utilisés tels que des rondelles anti-devissage et des écrous autofreinés.

L'extrémité supérieure de la partie proximale (1) est apte à être adaptée, par un moyen de fixation (B), à un outil de guidage porte-clou d'aide à l'insertion du dit clou centro-médullaire (100) dans un canal médullaire d'un os long d'un membre.

Ce dit outil est ainsi destiné à s'adapter à l'extrémité supérieure de la partie proximale (1) du clou centro-médullaire (100) grâce à un moyen de fixation (B), obturé après mise en place par une vis (6).

La partie distale (3) est apte à être introduite dans le canal médullaire.

La partie distale (3) est de forme soit semi-cylindrique, soit profilée, préférentiellement de forme profilée.

Notons que le clou centro-médullaire (100) est avantageusement de type court, adapté pour une réduction d'une fracture de la tête d'un os long d'un membre.

Précisons que le clou centro-médullaire (100) est avantageusement de type long, adapté pour une réduction d'une fracture du corps d'un os long d'un membre ou d'un allongement osseux.

La partie centrale (2) est selon certains modes de réalisation particuliers, incurvée selon un diamètre de giration ou incurvée selon un angle avec la partie proximale tel qu'illustré par le document US 5,472,444,

## Revendications

1. Clou centro-médullaire (100), d'un os long, comprenant un corps plein et au moins un orifice de fixation (4) apte à coopérer avec un organe de fixation (5), ledit orifice de fixation (4) comprenant un taraudage à au moins deux filets, **caractérisé en ce que** le taraudage est réalisé sur une partie du diamètre du clou centro-médullaire, à savoir que le taraudage est entouré latéralement de part et d'autre par des conduits non taraudés.

2. Clou centro-médullaire (100) selon la revendication 1, **caractérisé en ce que** les conduits non taraudés sont rectilignes sur au moins une partie.

3. Clou centro-médullaire (100) selon la revendication 1 ou 2, **caractérisé en ce que** le taraudage est centré sur le diamètre du clou centro-médullaire.

4. Clou centro-médullaire (100) selon l'une quelconque des revendications précédentes, **caractérisé en ce que** la partie du diamètre, comprenant le taraudage, est comprise entre 20 et 80 % du diamètre.

5. Clou centro-médullaire (100) selon l'une quelconque des revendications précédentes, **caractérisé en ce que** le taraudage est réalisé sur une partie du diamètre compris entre 20 et 60 %.

6. Clou centro-médullaire (100) selon l'une quelconque des revendications précédentes, **caractérisé en ce que** le taraudage est réalisé sur une partie du diamètre compris entre 20 et 40 %.

7. Clou centro-médullaire (100) selon l'une quelconque des revendications précédentes, **caractérisé en ce que** le taraudage est réalisé sur environs le tiers du diamètre.

8. Clou centro-médullaire (100) selon l'une quelconque des revendications précédentes, **caractérisé en ce que** l'orifice de fixation (4) comprend un taraudage à trois filets.

9. Clou centro-médullaire (100) selon l'une quelconque des revendications précédentes, **caractérisé en ce que** l'orifice de fixation (4) comprend un taraudage avec un pas axial (Pa) constant et un profil triangulaire.

10. Clou centro-médullaire (100) selon l'une quelconque des revendications précédentes, **caractérisé en ce que** la partie proximale (1) comprend au moins deux orifices de fixation (4) décalés angulairement les uns part à rapport aux autres selon un angle compris entre 20° et 50°, préférentiellement entre 30° et 40°.

11. Clou centro-médullaire (100) selon l'une quelconque des revendications précédentes, **caractérisé en ce que** l'orifice de fixation (4) comprend un pas hélicoïdal (Ph) compris entre 1 et 3 millimètre(s).

12. Clou centro-médullaire (100) selon l'une quelconque des revendications précédentes, **caractérisé en ce que** l'orifice de fixation (4) et/ou l'organe de fixation (5) comprend un moyen de blocage dudit organe de fixation (5).

## Patentansprüche

1. Intramedullärer Nagel (100) eines langen Knochens, der einen festen Körper und mindestens eine Befestigungsöffnung (4) aufweist, die dazu ausgelegt ist, mit einem Befestigungselement (5) zusammenzuwirken, wobei die Befestigungsöffnung (4) mindestens eine Gewindebohrung aufweist zwei Fäden, **dadurch gekennzeichnet, daß** der Abstich über einen Teil des Durchmessers des Intramedullärer Nagels gebildet ist, und zwar ist der Abstich seitlich beidseitig von nicht angezapften Rohren umgeben.

2. Intramedullärer Nagel (100) nach Anspruch 1, **dadurch gekennzeichnet, dass** die gewindelosen Leitungen zumindest über einen Teil geradlinig sind.

3. Intramedullärer Nagel (100) nach Anspruch 1 oder 2, **dadurch gekennzeichnet, dass** die Anbohrung auf den Durchmesser des intramedullärer Nagels zentriert ist.

4. Intramedullärer Nagel (100) nach einem der vorhergehenden Ansprüche, **dadurch gekennzeichnet, dass** der Teil des Durchmessers, der den Abstich aufweist, zwischen 20 und 80% des Durchmessers beträgt.

5. Intramedullärer Nagel (100) nach einem der vorhergehenden Ansprüche, **dadurch gekennzeichnet, dass** der Gewindebohrer auf einem Teil des Durchmessers zwischen 20 und 60% ausgebildet ist.

6. Intramedullärer Nagel (100) nach einem der vorhergehenden Ansprüche, **dadurch gekennzeichnet, dass** das Gewindeschneiden über einen Teilbereich des Durchmessers zwischen 20 und 40% ausgebildet ist.

7. Intramedullärer Nagel (100) nach einem der vorhergehenden Ansprüche, **dadurch gekennzeichnet, dass** der Gewindebohrer über etwa ein Drittel des Durchmessers ausgebildet ist.

8. Intramedullärer Nagel (100) nach einem der vorhergehenden Ansprüche, **dadurch gekennzeichnet, dass** die Fixierungsöffnung (4) eine Gewindebohrung mit drei Gewindegängen aufweist.

9. Intramedullärer Nagel (100) nach einem der vorhergehenden Ansprüche, **dadurch gekennzeichnet, dass** die Fixieröffnung (4) eine Gewindebohrung mit konstanter axialer Steigung (Pa) und einem dreieckigen Profil aufweist.

10. Intramedullärer Nagel (100) nach einem der vorhergehenden Ansprüche, **dadurch gekennzeichnet, dass** der proximale Abschnitt (1) mindestens zwei Befestigungslöcher (4) aufweist, die in einem Winkel zwischen 20 ° und 50 ° zueinander winkelversetzt sind °, vorzugsweise zwischen 30 ° und 40 °.

11. Intramedullärer Nagel (100) nach einem der vorhergehenden Ansprüche, **dadurch gekennzeichnet, dass** die Fixierungsöffnung (4) eine Helixsteigung (Ph) zwischen 1 und 3 mm aufweist.

12. Intramedullärer Nagel (100) nach einem der vorhergehenden Ansprüche, **dadurch gekennzeichnet, dass** die Befestigungsöffnung (4) und / oder das Befestigungselement (5) eine Verriegelungseinrichtung zum Verriegeln des Befestigungselements (5) aufweist.

## Claims

1. Intramedullary nail or rod (100) of a long bone, comprising a solid body and at least one fixing opening (4) adapted to cooperate with a fixing member (5), said fixing opening (4) comprising a tapping with at least two threads, **characterized in that** the tapping is formed over a portion of the diameter of the intramedullary nail, namely the tapping is laterally surrounded on both sides by non-tapped conduits.

2. Intramedullary nail (100) according to claim 1, **characterized in that** the unthreaded conduits are rectilinear over at least a portion.

3. Intramedullary nail (100) according to claim 1 or 2, **characterized in that** the tapping is centered on the diameter of the intramedullary nail.

4. Intramedullary nail (100) according to any one of the preceding claims, **characterized in that** the portion of the diameter comprising the tapping is between 20 and 80% of the diameter.

5. Intramedullary nail (100) according to any one of the preceding claims, **characterized in that** the tapping is formed on a portion of the diameter between 20 and 60%.

6. Intramedullary nail (100) according to any one of the preceding claims, **characterized in that** the tapping is formed over a portion of the diameter between 20 and 40%.

7. Intramedullary nail (100) according to any one of the preceding claims, **characterized in that** the tapping is formed over about one third of the diameter.

8. Intramedullary nail (100) according to any one of the preceding claims, **characterized in that** the fixing opening (4) comprises a tapping with three threads.

9. Intramedullary nail (100) according to any one of the preceding claims, **characterized in that** the fixing opening (4) comprises a tapping with a constant axial pitch (Pa) and a triangular profile.

10. Intramedullary nail (100) according to any one of the preceding claims, **characterized in that** the proximal portion (1) comprises at least two fixing holes (4) angularly offset one relative to the other according to an angle between 20° and 50°, preferably between 30° and 40°.

11. Intramedullary nail (100) according to any one of the preceding claims, **characterized in that** the fixing opening (4) comprises a helical pitch (Ph) between 1 and 3 millimeters.

12. Intramedullary nail (100) according to any one of the preceding claims, **characterized in that** the fixing opening (4) and / or the fixing member (5) comprises a locking means for locking said fixing member (5).
